# EUROPEAN PATENT APPLICATION

(11) **EP 1 349 103 A2**
(43) Date of publication of application: **01.10.2003**
(21) Application number: 03251850.8
(22) Date of filing: 25.03.2003
(51) Int. Cl.: G06F 19/00

(54) **Apparatus and method for finding genes associated with diseases**

(30) Priority: 26.03.2002 US 107377
(71) Applicant: Hewlett-Packard Company, Palo Alto, CA 94304 (US)
(72) Inventor: Adamic, Lada A., Los Altos, CA 94024 (US); Huberman, Bernardo A., Palo Alto, CA 94301 (US); Wilkinson, Dennis M., Los Altos, CA 94022 (US); Adar, Eytan, Palo Alto, CA 94303 (US)
(74) Representative: Tollett, Ian

(57) **Abstract**

A method (200) of finding genes associated with a diseases, includes: finding (205) all potential gene symbols; folding (210) at least one alias into official gene symbols; and computing (215) the relevance of each official symbol to the disease. The method may further include, eliminating (220) non-gene symbols by use of contextual clues.

## Description

The present invention relates generally to an apparatus and method for finding genes associated with diseases.

Biological and medical literature (including written papers, books, studies, and/or reports) are now increasingly being electronically published or stored in electronic media. For example, MedLine <http://www4.ncbi.nlm.nih.gov/PubMed/> is an electronic database containing over 11 million citations (titles and/or abstracts) covering publications since 1960 as compiled by the National Library of Medicine. By utilizing these collections of information, it may be possible to discover novel gene expression pathways that can help in the development of new or improved methods for treating particular human diseases.

However, a researcher having access to this electronic collection of information is also required to be able to identify and filter out the irrelevant articles. For example, the word "leukemia" appears in over 22,177 articles in MedLine. Thus, a great amount of effort and time would be required to manually extract useful information embedded in such a large volume of stored data.

Various methods are available for automated extraction of biomedical knowledge However, these methods do not sufficiently reduce the amount of retrieved articles that are irrelevant to the topic being searched. For example, these current methods would result in the retrieval of many citations that are false positives because these methods are unable to disambiguate the relevant citations that are stored in an electronic database. Therefore, the current technologies are limited to particular capabilities and suffer from various constraints.

In an embodiment of the present invention, a method of finding genes associated with a disease, includes: finding all potential gene symbols in articles (or titles/abstracts) in a database (or some repository); folding any aliases into official gene symbols; and computing the relevance of each official symbol to the disease. The method may further include, eliminating non-gene symbols by use of contextual clues.

In another embodiment, an apparatus for finding genes associated with a disease, includes: a database for storing information; and a server coupled to the database and configured to find all potential gene symbols in the stored information, to fold at least one alias into official gene symbols, and to compute the relevance of each official symbol to the disease. The server may be configured to eliminate non-gene symbols by use of contextual clues.

These and other features of an embodiment of the present invention will be readily apparent to persons of ordinary skill in the art upon reading the entirety of this disclosure, which includes the accompanying drawings and claims.

Non-limiting and non-exhaustive embodiments of the present invention are described with reference to the following figures, wherein like reference numerals refer to like parts throughout the various views unless otherwise specified.
Figure 1 is a block diagram of an apparatus in accordance with an embodiment of the invention.
Figure 2 is a flowchart that shows an entire procedure for finding relevant genes, in accordance with an embodiment of the invention.
Figure 3 is a flowchart that shows a detailed account of the process of folding aliases into official symbols, in accordance with an embodiment of the invention.
Figure 4A is a flowchart that shows a method 380 for measurement of the relevance of individual genes to a disease, in accordance with an embodiment of the invention.
Figure 4B is a flowchart that shows a method for measurement of the relevance of gene pairs to a disease, in accordance with an embodiment of the invention.
Figure 4C is a graph showing a distribution of correlation strengths between leukemia and various genes mentioned with leukemia in articles.
Figure 5 is a flowchart that shows a detailed account of the disambiguation process in order to accept or reject a symbol as a gene symbol, in accordance with an embodiment of the invention.

In the description herein, numerous specific details are provided, such as examples of components and/or methods, to provide a thorough understanding of embodiments of the invention. One skilled in the relevant art will recognize, however, that an embodiment of the invention can be practiced without one or more of the specific details, or with other apparatus, systems, methods, components, materials, parts, and/or the like. In other instances, well-known structures, materials, or operations are not shown or described in detail to avoid obscuring aspects of embodiments the invention.

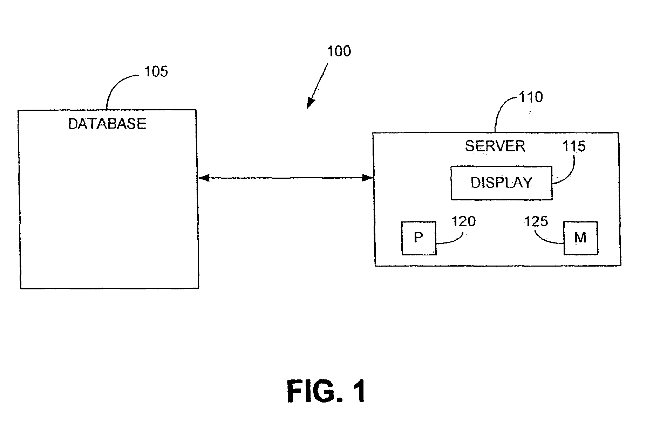

Figure 1 is a block diagram of an apparatus 100 in accordance with an embodiment of the invention. The apparatus 100 includes a database 105 that can store, for example, medical and/or scientific records or literature in electronic form. As an example, the database 105 is the MedLine database, although other suitable databases that store medical and/or scientific records may be used in Figure 1. The apparatus 100 also includes a server 110 that can access and/or retrieve information that is stored in the database 105. The server 110 may be, for example, a workstation, personal computer, notebook, laptop, a suitable portable computing device, or another type of computing device. The information accessed or retrieved by the server 110 may be displayed in a display portion 115 which may be integrated with the server 110 or separately coupled to the server 110. In one embodiment, the server 110 also includes a processor 120 that can execute a module or software 125 to enable an automated method of finding genes associated with diseases, as described in additional detail below. In one embodiment, as described further below, the automated method automatically extracts mentions of gene names from the database 105, specifically in those articles mentioning specific diseases or gene pathways. The method permits, for example, a physician or researcher to quickly obtain information about which particular gene(s) may be responsible for and/or is associated with a given disease. This is particularly useful for a physician or researcher, if he or she is instead an expert in another disease or field.

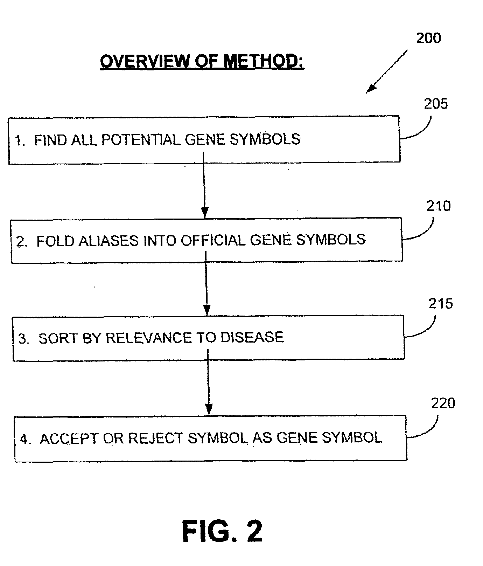

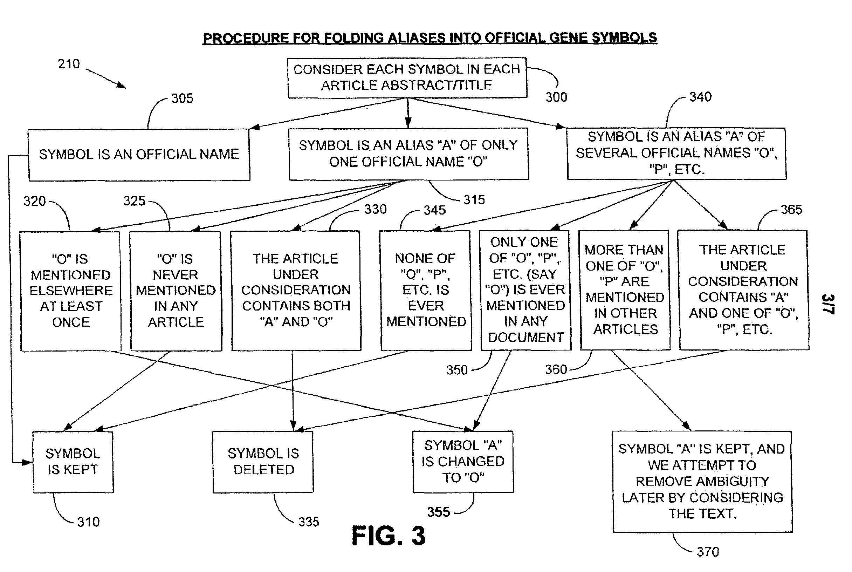

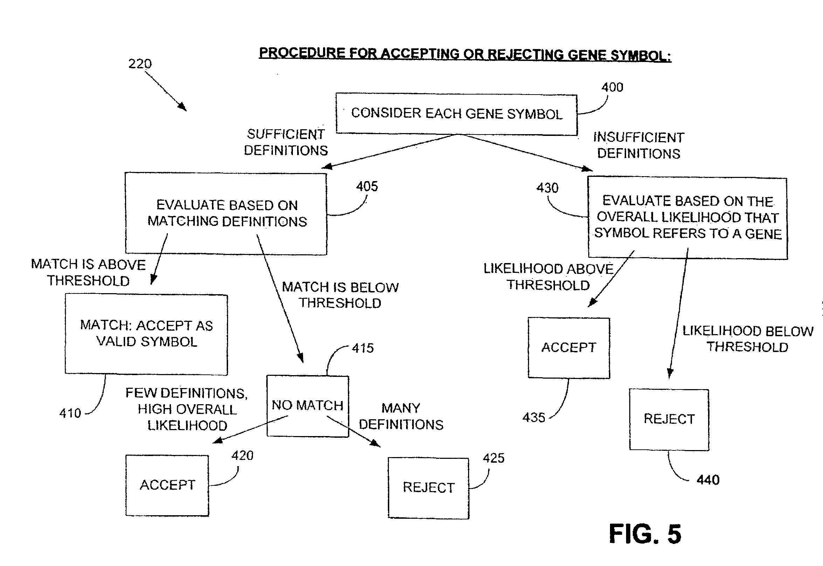

Figure 2 is a flowchart that shows an entire procedure 200 for finding relevant genes, in accordance with an embodiment of the invention. As discussed in detail below, the method 200 includes extracting (205) gene symbols (i.e., finding all potential gene symbols), folding (210) aliases into official symbols, and computing (215) the relevance of each official symbol to the disease. ln an embodiment, the method 200 further includes accepting/eliminating (220) a symbol as a gene symbol by using contextual clues, such as whether the symbol has an overall likelihood to be representing a gene or whether its accompanying definitions match the official or alias gene names. As also discussed below, Figure 3 is a flowchart that shows a detailed account of the process of folding (210) aliases into official symbols, and Figure 5 is a flowchart that shows a detailed account of the disambiguation process (220) in order to accept or reject a symbol as a gene symbol.

In describing the process of method 200, Medline is used as an example of the database 105 (Figure 1) that is searched by the server 110 for information. However, other suitable databases may be used instead of Medline. Additionally, it is also noted that in the below text, particular names are used to identify the genes, aliases, parameters, and/or other items (e.g., PMID, OS, or MID1). These particular names are only provided as some possible examples to identify genes, aliases, parameters, and/or other items, and other names may be used to identify the genes, aliases, parameters, and/or other items shown in the drawings and/or discussed in the below text.

### Gene Frequencies in MedLine (or other database)

In procedure or action (205) in Figure 2, the method 200 performs an automated search of the abstract and title of the Medline records to produce a "AMID/gene list" which, for each document, is identified by a unique PMID number, and lists the different HUGO, OMIM, and LocusLink gene symbols that occurred in the abstract or title. In one embodiment, the procedure (205) does not search for the full name of each gene, only its symbol, and the procedure (205) also does not count how many times a particular symbol occurs in each article. The procedure (205) just determines whether the symbol occurs in the abstract or title.

Additionally, the procedure (205) may record the publication date of each article and may determine whether the article's abstract or title contained a word or words pertaining to a particular disease or gene expression pathway. For example, if the search were focusing on the leukemia disease, then a search is made for the words "leukemia" or "leukaemia" in the Medline database. The method of procedure (205) can then isolate the lists of genes in those articles pertaining to leukemia.

### Coping with alias symbols

It is noted that gene names can be represented by gene symbols (see, e.g., <http://www.gene.ucl.ac.uk/public-files/nomen/ens2.txt>) and aliases (see, e.g., <http://www.gene.ucl.ac.uk/public-files/nomen/ens3.txt>) typically listed by three (3) online gene databases: HUGO (Human Genome Organization), OMIM (Online Mendelian Inheritance in Man), and LocusLink (an online database of gene loci). The use of gene symbols and/or aliases for a given gene name adds to the current difficulty in distinguishing between relevant and irrelevant articles in databases searches for that given gene, since a given gene may have multiple identifiers.

The process of identifying gene mentions by the occurrence of gene symbols is also naturally error prone. A gene symbol can coincide with another common acronym, or with an acronym constructed by the author for the purposes of the article. For example, an author might have used the acronym CGH to mean "comparative genomic hybridization", while CGH might be recorded as an alias for the gene HTC2. As long as the errors are equally likely to occur within the focus set as in all of Medline, the embodiments of algorithms (as disclosed herein) will not be misled by the errors.

However, when an acronym is specific to a focus set, and yet does not represent a gene, further processing is needed to disambiguate the meaning of the acronym. Applicants present their approach or method to dealing with this problem by use of a procedure (220) as illustrated in Figure 5 and discussed in corresponding text below.

Even when a word in a document is being used to denote a gene, frequently the word is an alias rather than an approved gene name. Thus, in an embodiment of the invention, a post-processing procedure (210) may be required to match an alias to a particular gene, as shown by the flowchart in Figure 3.

To match an alias to a particular gene, a count is performed all occurrences of gene names (official symbols and aliases) within the entire article set and within the focus subset. Here, "entire article set" might refer to the Medline database, while "focus subset" might pertain to only those articles whose titles or abstracts contain the word "leukemia", for example. For each alias occurrence, the procedure (210) adds to the count of both the alias and the official gene or genes it represented. For example, if the symbol OS, an alias for MID1, occurred in 49 articles, while MID1 occurred in 3, MID1 would have a count of 52. The procedure (210) keeps track of the fact that 49 of the-counts for MID1 originated with OS to be able to relate back to the articles and to modify the document gene lists as described below. Because OS frequently stands for "overall survival", it is important to keep track of its contribution to MID1's counts, as MID1 could otherwise be incorrectly related to a disease.

In procedure (210), there is a modification of the PMID/gene lists for the entire set and the focus subset to account for alias symbols. For each alias symbol, there are typically four possibilities:
1. The alias symbol represents only one official symbol, and the official symbol appears independently (that is, the count of the official symbol was greater than its alias' count). For this case, the procedure (210) replaces all mentions of the alias in question in the PMID/ gene lists with the official symbol. )
2. The alias symbol represents more than one official symbol, but only one of these official symbols occurs independently. For this second case, the procedure (210) replaces the alias symbol with the official symbol which had counts.
3. The alias symbol represents one or more official symbols, but none of these official symbols ever occurred independently within the subset. For this case, the procedure (210) keeps the alias symbol. For this case, the reasoning was that the official symbol was obviously not widely accepted by researchers in the area of our focus, so it would be more reasonable to refer only to the alias symbol.
4. The alias symbol represents more than one official symbol, and at least two of these official symbols have independent occurrences within the subset. In this case, the procedure (210) could not decide without syntactic analysis of the abstract or title text, which official symbol that the alias represented in each particular case. Fortunately, there were few of these instances.

In all cases, the procedure (210) keeps the information about where the counts originally came from and indicates this information in our results. For example, let's say our results implicate an obscure official symbol, which almost always appeared as the well-used alias symbol, in some disease. The original counts would show the user that 95% of the time that the gene was mentioned in connection with the disease, it was mentioned as the alias and not as the obscure official symbol, hopefully mitigating any confusion.

The procedure (210) in Figure 3 is now discussed in step-by-step detail. Each alias symbol is considered (310) in the abstract and title of an article. If the alias symbol is an official name (procedure 305), then procedure (310) keeps the alias symbol.

If the alias symbol is, for example, an alias "A" of only one official name, for example, "O" (procedure 315), the various following conditions are considered. If "O" is mentioned elsewhere at least once in an article (procedure 320), then the alias symbol is deleted (335). If "O" is never mentioned in any article (procedure 325), then the symbol "A" is changed (335) to "O". If the article under consideration contains both "A" and "O" (procedure 330), then the symbol is deleted (335).

If the alias symbol is, for example, an alias "A" of several official names "O", "P", etc. (procedure 340), then the various following conditions are considered. If none of "O", "P", etc. is ever mentioned (procedure 345), then the alias symbol is kept (310). If only one of "O", "P", etc (say "O") is ever mentioned in any document (procedure 350), then the symbol "A" is changed (355) to "O". If more than one of "O", "P" are mentioned in other articles (procedure 360), then the symbol "A" is kept, and an attempt to remove ambiguity is later performed by considering the text (procedure 370). If the article under consideration contains "A" and one of "O", "P", etc. (procedure 365), then the symbol is deleted (355).

### Counting n-tuple occurrences

From the simplified PMID/gene lists, the method 200 can create data sets containing counts for each n-tuple of genes. For example, the Medline article with PMID number 8563753 discusses human myeloid leukeima and mentions the genes NUP98, HOXA9, and NUP214. So from this article, we obtained one count for each of these three genes, one count each for the pairs NUP98--NUP214, NUP98--HOXA9, and NUP214--HOXA9, as well as one count for the triple containing all three genes NUP98--HOXA9--NUP214.

In the method (200), we initially created data sets for individual gene occurrences (post-modification for aliases), gene pairs and gene triples.

### Measuring the relevance of individual genes

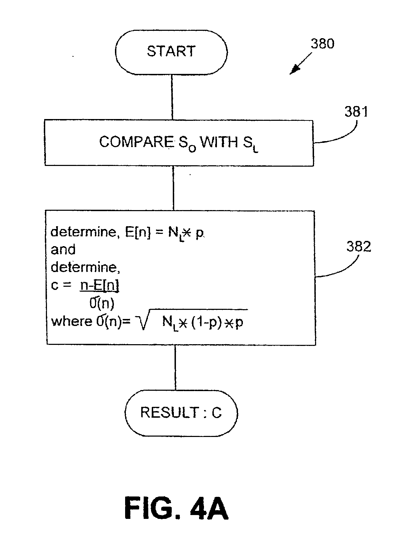

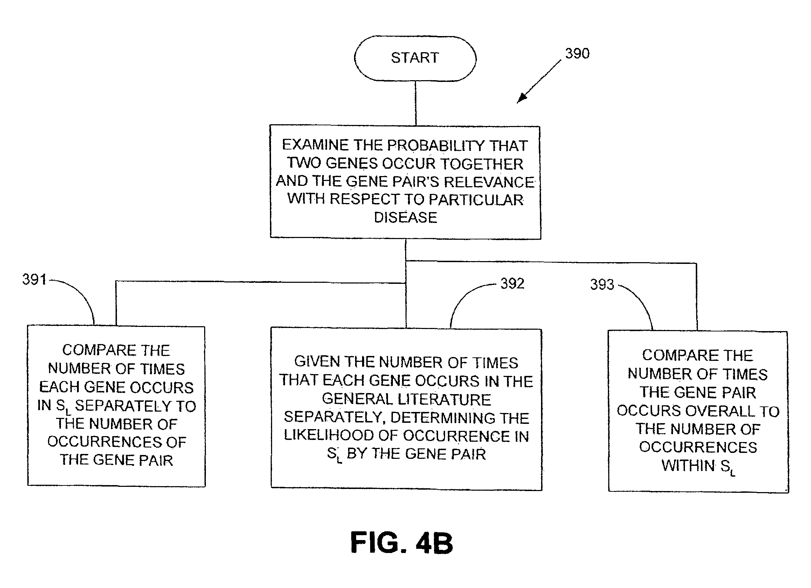

A detailed discussion is now made on the procedure (215) for sorting the relevance of genes to a disease. A discussion is first made on a method 380 (Figure 4A) for measurement of the relevance of individual genes to a disease and then a discussion is made on a method 390 (Figure 4B) for measurement of the relevance of gene pairs to a disease.

As shown in Figure 4A, a comparison (381) is made for the frequency of occurrence of a gene name in the set of all Medline articles (S₀) to the frequency with which the gene occurred in the focus subset (*S*_{*L*}). The focus subset (*S*_{*L*}) which pertains to a particular disease or gene expression pathway. The intuition is that if the gene A is more frequently mentioned in the documents which contain the word, "leukemia" than in the overall set of articles in a database, then these is a chance that gene A has been specifically linked to leukemia in the literature.

Focusing on leukemia, consider, for example, the gene MLL, which our measure shows to be most tied to leukemia. The official HUGO symbol MLL stands for myeloid/lymphoid or mixed-lineage leukemia (trithorax (Drosophila) homolog). The gene MLL aliases include HTRX1, HRX, and ALL-1.

The symbol MLL occurs in 548 of the 39710 articles mentioning leukemia and containing a gene symbol, and 633 times in the 2 million articles containing gene symbols. If we put aside for the moment that the name MLL itself states the relationship of the gene to leukemia, we could we use the above data to determine how strong the relationship is between MLL and leukemia.

We do this by measuring (382) how unlikely it would be to see the number of gene mentions in *S*_{*L*}, given how frequently the gene is mentioned overall. Let's represent all the MLL documents with black balls, and all other documents as white balls. If we assume that there is no correlation between MLL and leukemia, then the distribution of the number of MLL documents in *S*_{*L*} (the number of black balls drawn) is given by the Binomial distribution.

The expected number of MLL documents is given by, *E*[*n*_{*MLL*}]=*N*_{*L*} **p*_{*MLL*}, where *p*_{*MLL*} is the probability of drawing a black ball or 0.0003, and *N*_{*L*} is the number of documents in the *S*_{*L*} (the number of draws from the urn). The standard deviation is given by σ(*n*_{*MLL*}) = $\sqrt{{\text{N}}_{\text{L}} \text{*(1} {\text{-p}}_{\text{MLL}} \text{)} {\text{*p}}_{\text{MLL}} \text{.}}$Also, *n*_{*MLL*} is the number of observed documents (in this case, in the leukemia set) with MLL. We measure the strength of the relationship (*c*_{*MLL*}) between MLL and leukemia by measuring how much the observed number of MLL documents (black balls) deviates from the expected number had the draw been random, as shown in equation (1).${\text{c}}_{\text{MLL}} \text{=} \frac{{\text{n}}_{\text{MLL}} \text{-} \text{E} \text{[} {\text{n}}_{\text{MLL}} \text{]}}{\text{σ} \text{(} {\text{n}}_{\text{MLL}} \text{)}}$ We find that *c*_{*MLL*} *=* 133.5, which is a very high value. We have used the normal approximation to the binomial distribution, valid in the case of large N. Using the normal distribution we can also find that the probability that 548 or more MLL documents are found among a random draw of 39710 documents is less than 10⁻¹⁶. Our finding is consistent with a summary from the *Atlas of Genetics and Cytogenetics in Oncology and Haematology* <http://www.infobiogen.fr/services/chromcancer/index.html> "MLL is implicated in at least 10 % of acute leukemias (AL) of various types".

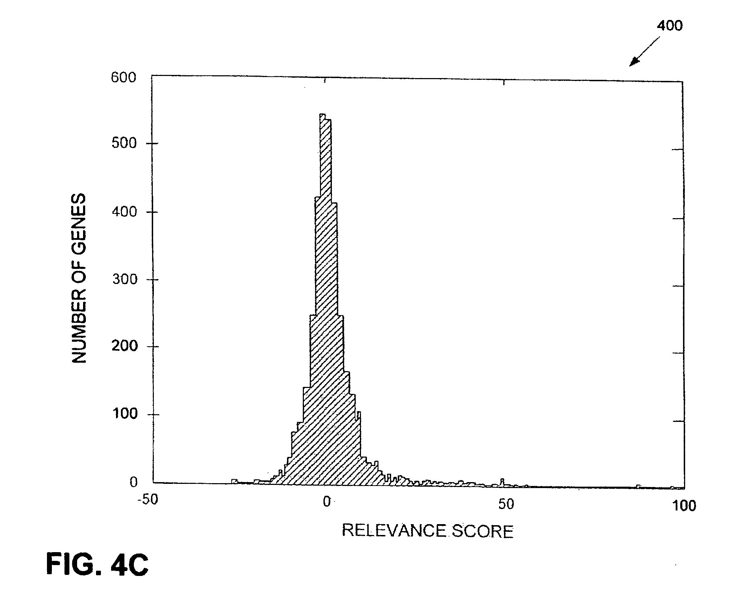

Most genes, however, show little or negative correlation with leukemia as demonstrated in the distribution 400 in Figure 4C. The distribution 400 shows the values of *c*_{*MLL*} for all genes which occur in *S*_{*L*}. In other words, the distribution 400 shows the correlation strengths between leukemia and various genes mentioned with leukemia in articles. Figure 4C lacks those genes which occur, in the database, but do not occur in *S*_{*L*} at all. They would populate the negative correlation side of Figure 4C.

Table 1 shows an example of the output of the algorithm identifying relevant breast cancer genes. The results shown in Table I may be shown, for example, in the display 115 of the server 110 (Figure 1). Note that the output shown in Table 1 makes use of a method for disambiguation of symbols (procedure 220), as described below in additional detail. Symbols are shown in order of relevance given by the function given in Equation (1) above. They are subsequently evaluated for their potential to be gene symbols. Official gene symbols are shown in blue (row(1)-row(11)), while alias symbols that can be mapped to more than one official gene symbol are shown in green (e.g., rows (2a)-(5a)). All aliases which occur at least once are listed along with the official symbol. The yellow hue of the box is more saturated for higher r_{G} (the symbol is more likely to be a gene). If the majority of the symbols is accepted as a gene symbol, the gene as a whole is rated as relevant to breast cancer. In this way, an embodiment of the invention permits us to find several important breast cancer genes such as BRCA1, ERBB2, ESR1, BRCA2, PGR, EGFR, TFF1, TP53, and CEACAM5. At the same time we are able to eliminate non-gene acronyms: MB (a symbol contained in a cell line name), FAC and CAF (5-fluorouracil, Adriamycin, cyclophosphamide chemotherapy), SLN (sentinel lymph node), OS (overall survival), DCC (dextran coated charcoal), TNM (tumor node metastasis). We were also able to disambiguate the symbol ER to ESR1 (estrogen receptor 1) and even though ER can also be an alias for EREG (epiregulin). The disambiguation procedure (220) is described in detail below with reference to Figure 5 and associated text.

If and when the algorithm does make mistakes, it is in rare cases where the symbol is absent from the gene alias databases. An error can also occur when the gene symbol is genuine but overlaps with another common acronym and has no supporting definitions occurring in text. For example the FOR alias for the WWOX gene occurs 139 times in articles mentioning breast cancer. However, it is never accompanied by a definition, and so is rejected as a gene symbol based on the overall likelihood that FOR is a gene symbol which is only about 10%. The WWOX gene symbol itself would nevertheless be identified as relevant, as it occurs 4 out of 5 with the words "breast cancer/tumor".

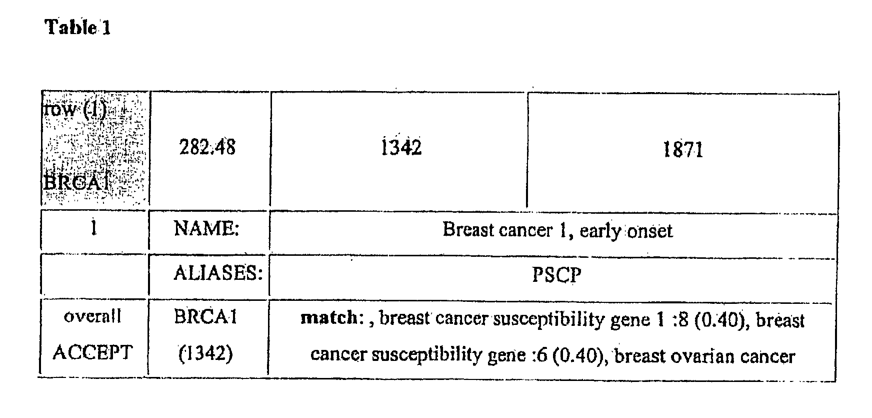

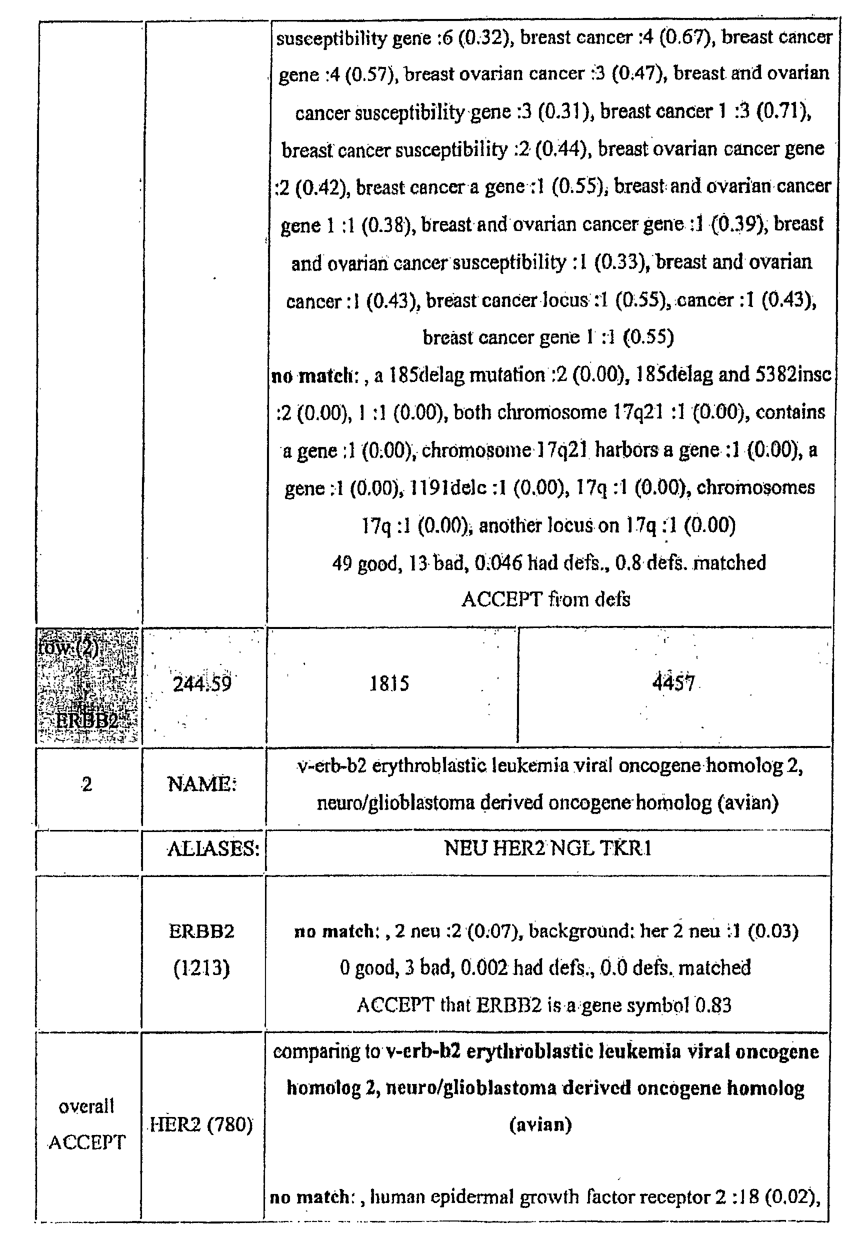

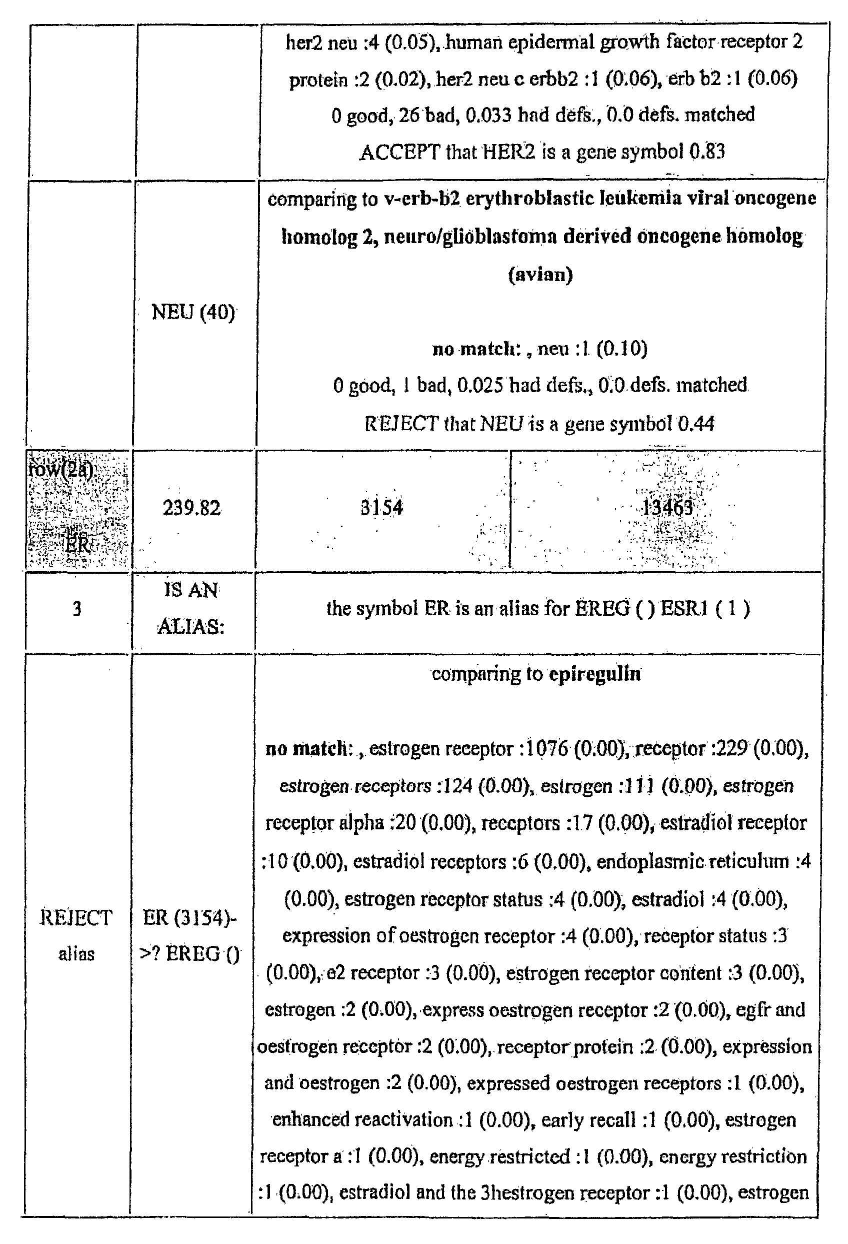

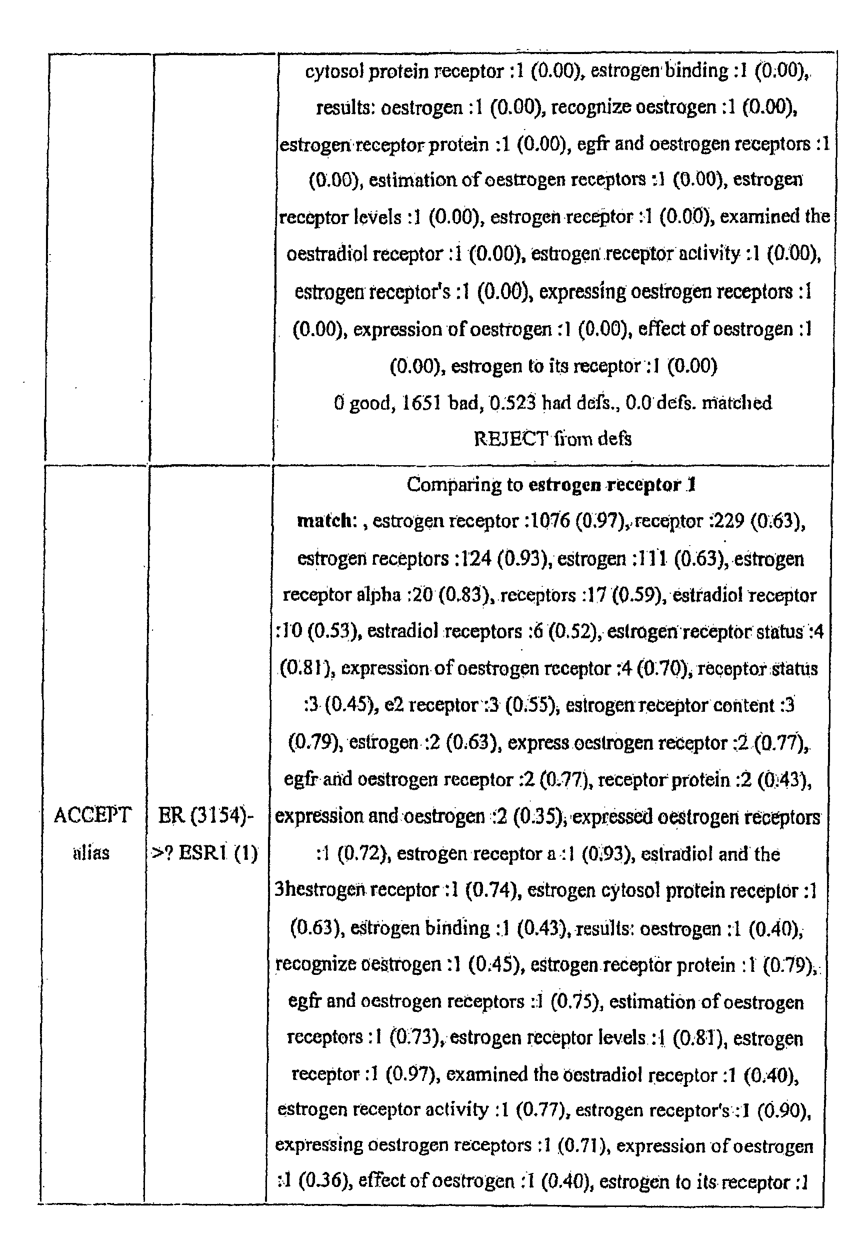

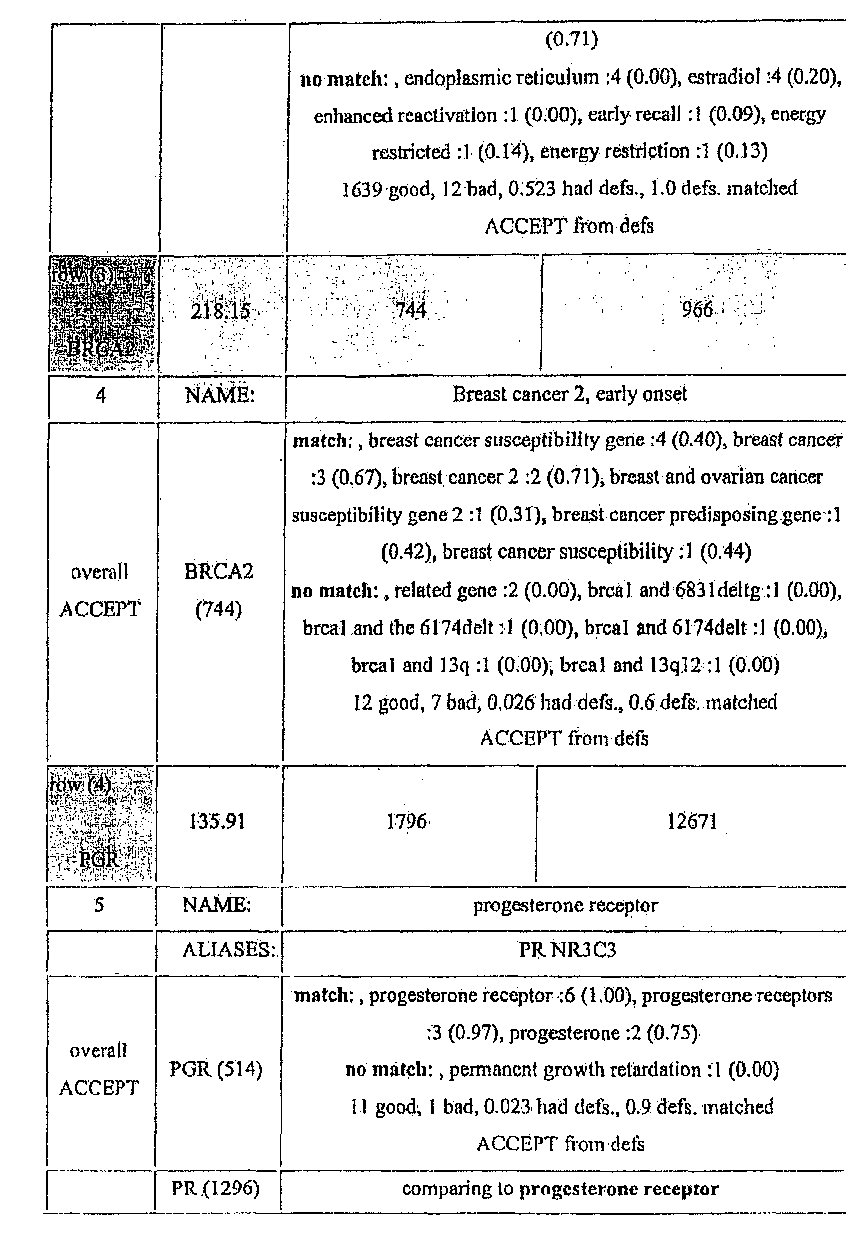

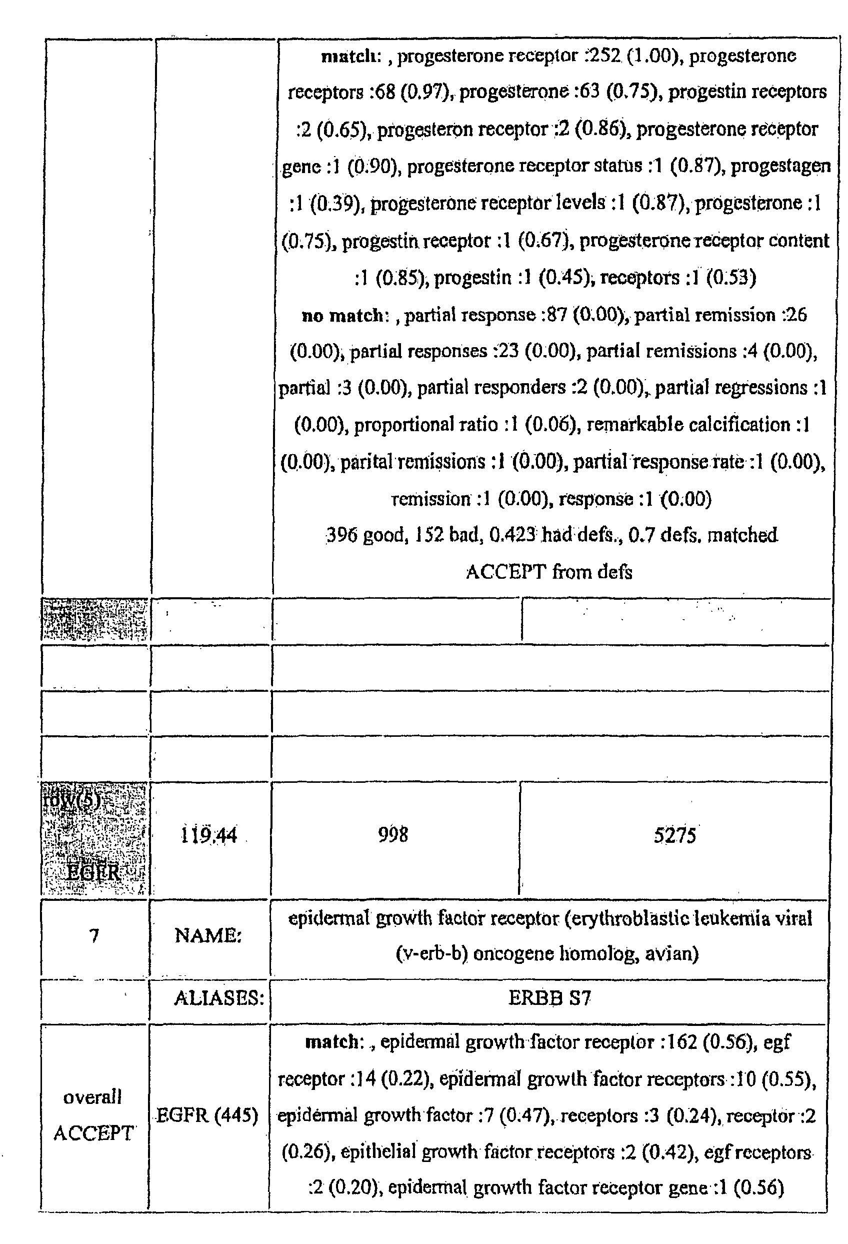

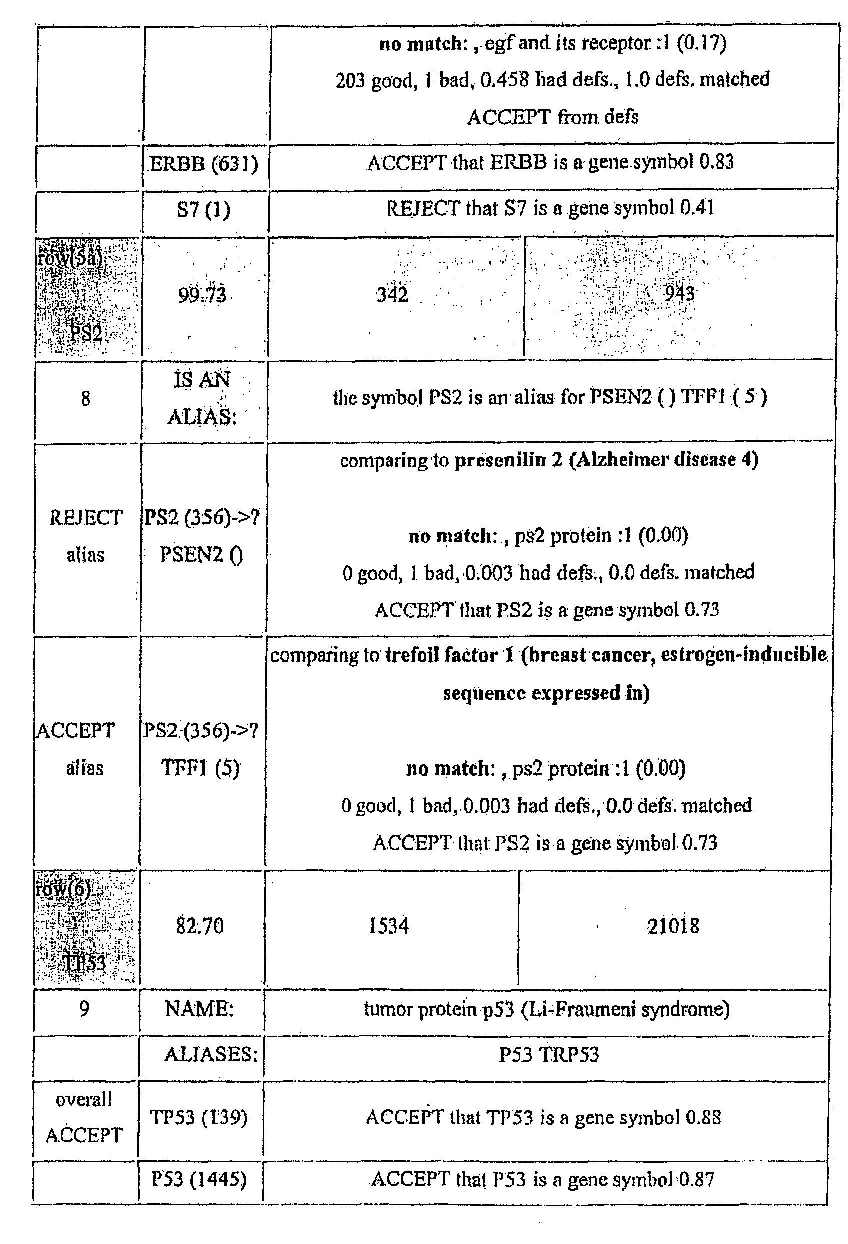

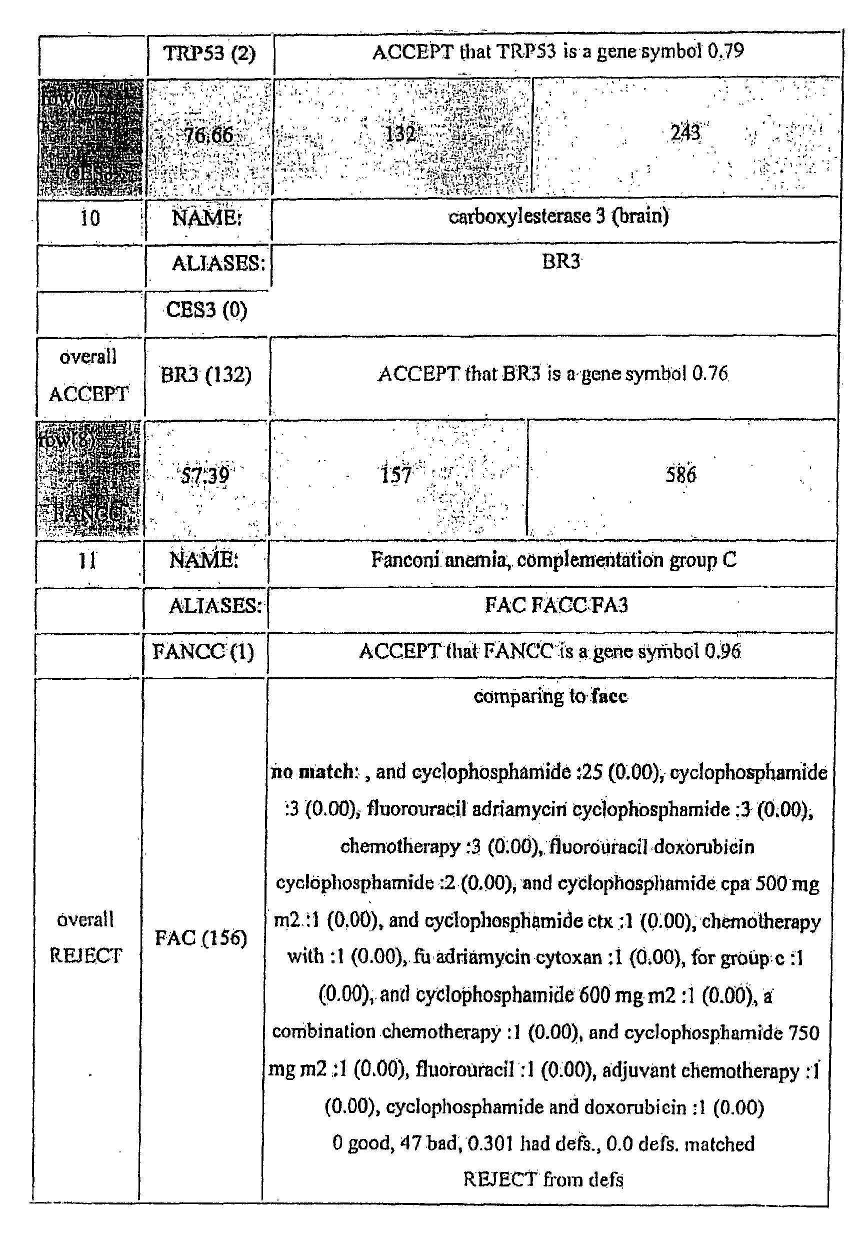

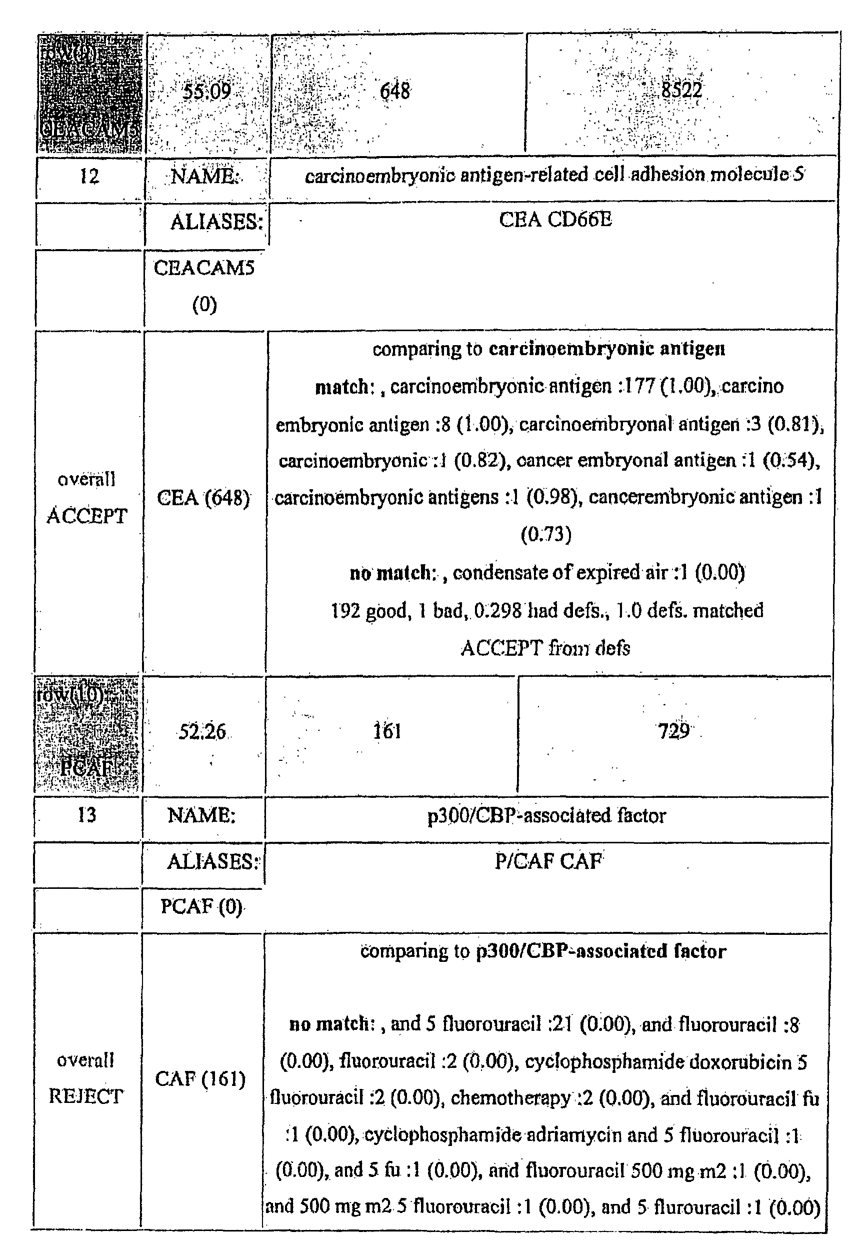

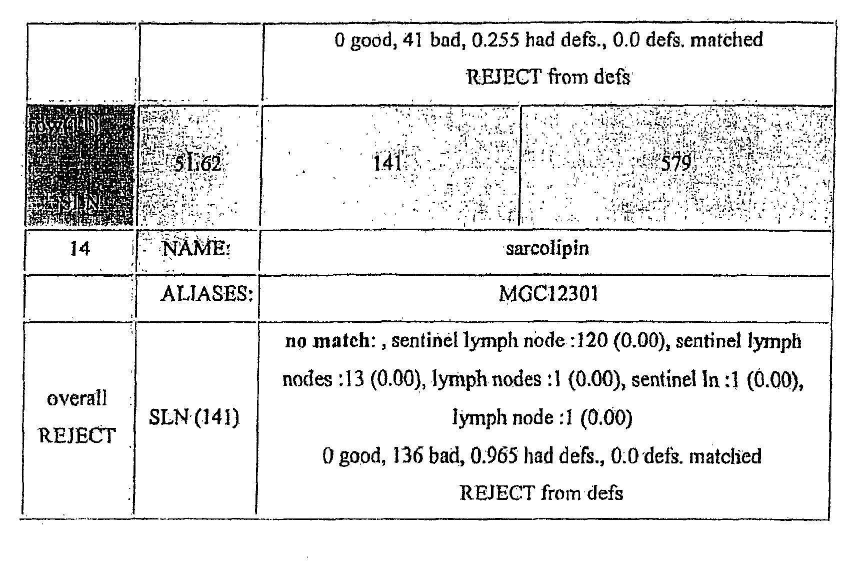

### Relevance of gene pairs

As shown in the method 390 in Figure 4B, next we examined the probability that two genes occur together and the pair's relevance with respect to a particular gene. There are three possible routes.
a) Compare the number of times each gene occurs in *S*_{*L*} separately to the number of occurrences together (procedure 391 in Figure 4B). What is the likelihood that they occur together, i.e., is there a possibility that genes predominantly act together with respect to leukemia, or are their effects uncorrelated?
b) Given the number of times each gene occurs in the general literature separately, what is the likelihood that they occur together in *S*_{*L*}? (procedure 392 in Figure 4B).
c) Compare the number of times the pair occurs overall to the number of occurrences within *S*_{*L*} (procedure 392 in Figure 4B). This is analogous to the above calculation of the value of individual genes, and measures the relevance of pairs. In method (a), we Let p_{A} (p_{B}) be the fraction of documents with gene A(B) in *S*_{*L*}*.* In method (b) we let p_{A} (p_{B}) be the fraction of documents with gene A(B) in the entire document collection. Then if A and B are uncorrelated, the probability of finding them together is p_{AB}=p_{A}*p_{B}. From here on, we proceed just as we did for the link of a single gene to leukemia. Take for example the two genes CBFB and MYH11, which have an unusually high complementarity. CBFB occurs 44 times in *S*_{*L*} and MYH11 occurs 74 times, yet a full 28 of those occurrences are joint. The probability of this occurring is very small, and we obtain a complementarity score of 91.54 given by${\text{c}}_{\text{AB}} \text{=} \frac{{\text{n}}_{\text{AB}} \text{-} \text{E} \text{[} {\text{n}}_{\text{AB}} \text{]}}{\text{σ(} {\text{n}}_{\text{AB}} \text{)}} \text{=} \frac{{\text{n}}_{\text{AB}} \text{-} {\text{N}}_{\text{L}} {\text{*P}}_{\text{AB}}}{\sqrt{{\text{N}}_{\text{L}} \text{*(1-} {\text{p}}_{\text{AB}} \text{)*} {\text{p}}_{\text{AB}}}} \text{=91.54}$

Method (b) uses the probabilities of A and B occurring in the entire document collection. This means that most pairs of genes that were individually relevant to *S*_{*L*} will appear positively correlated simply because they occur more frequently in *S*_{*L*}, increasing the chance that they occur together in *S*_{*L*}. Hence, method (a) is preferable to (b) in determining whether A and B act together with regard to *S*_{*L*}*.*

Method (c) can be used to measure the relevance of a gene pair to a disease, just as one can measure the relevance of a single gene. If a gene pair occurs more frequently in *S*_{*L*} than in the entire document collection, then the pair is considered relevant to *S*_{*L*}*.* Using method c), we find that the CBFB-MYH11 pair occurs 28 times with leukemia, and 32 times overall, giving the pair a relevance score of 32.49 to leukemia.

Searching through the literature we find why CBFB and MYH11 are complementary to such an extent: "In human acute myeloid leukemia samples with chromosome 16 inversion, a fusion gene *CBFB-MYH11* is created and expressed. This novel gene includes most of the CBFB gene, a hematopoietic transcription factor, and the last half of MYH11" <http://www.umassmed.edu/pgfe/faculty/castilla.cfm>.

We find that genes located on the same chromosome are frequently studied together, which may or may not indicate an interesting gene interaction.

### Disambiguating gene symbols

When attempting to extract gene symbols from text, we face the problem of polysemy - the use of one symbol to refer to several terms. Ideally, we would like to know whether a symbol refers to a gene in order to correctly match genes to particular diseases or conditions. As shown in the method 220 in Figure 5, each gene symbol is -considered (400). The method 220 tackles the problem from two directions: calculating an overall likelihood that the symbol represents a gene (see procedure 430), and using specific cues from the text to verify that an individual title or abstract is referring to a particular gene (see procedure 405).

The method 220 calculates the likelihood that a symbol represents a gene by comparing the number of article titles and abstracts containing the symbol as well as words such as "gene","DNA", "inhibit","express", to the total number of articles in which the symbol occurs. The higher the value of the ratio r_{G}, the greater the likelihood that any given instance of the symbol is a gene reference. Thus, if the ratio r_{G} is above a threshold, then the method 220 can accept (435) the symbol as a gene reference. Typically, the threshold may beset to approximately 0.5. Otherwise, ratio r_{G} is below a threshold, then the method 220 can reject (440) the symbol as a gene reference.

While using r_{G} alone can be useful for positively identifying gene symbols with little ambiguity (i.e., the symbol is almost always used to refer to a gene), additional information may be needed to disambiguate symbols with multiple meanings. For example, the symbol DCC, used to denote the "deleted in colon cancer" gene, also occurs in the Medline abstracts as an abbreviation for "dextran coated charcoal", "dicyclohexylcarbodiimide", "day care center" and many other concepts. Its r_{G} is only 0.46, which places it below our threshold of 0.5. This information alone does not allow us to judge with certainty whether the symbol DCC refers to the gene in any given article.

Fortunately, authors sometimes offer on first mention a definition followed by the symbol itself in parenthesis. In procedure (405), the method 220 extracts the words preceding the parentheses and selects those most likely to form a definition, and then compares the definitions with the official gene name or names associated with an alias, if available. It is typically necessary for this operation to be fuzzy as definitions are not always exact matches. For example, one author may define the symbol ER as "estrogen receptor" (an exact match for the definition) while another may define it as "estrogen receptors." To support this variability the algorithm used attempts to break definitions into smaller components and compare the overlap of those to the initial definition. Specifically, the technique used is the deconstruction of definitions into n-grams, or substrings of length n. The 3-grams for "estrogen receptor," for example, are: est, str, tro, rog, etc. The power of such a technique is that it extracts "root" meanings from terms that are impossible to determine by direct comparison. For example, "estradiol receptor" and "estrogen receptor" are basically the same thing, but only a technique such as n-grams will be able to determine this. The distance between the official definition and the proposed definition is:$\text{similarity} \text{=} \frac{\text{∥} \text{A∩B} \text{∥+1}}{\sqrt{\text{∥} \text{A} \text{∥+1}} \text{*} \sqrt{\text{∥} \text{B} \text{∥+1}}}$ Where the numerator is the number of intersecting n-grams between the true definition, A, and the proposed definition, B. The denominator a normalization factor based on the number of n-grams in both definitions. The resulting similarity value is then compared to a threshold. If the match is above a threshold, then the symbol is accepted (410) as a valid gene symbol. If the match is below the threshold, then if there are few definitions, the symbol is accepted (420) as a valid gene symbol because this condition sets forth there is a high overall likelihood that the symbol is valid. In contrast, if there are many definitions, then the symbol is rejected (425) as a valid gene symbol.

As an example, Table 2 lists an evaluation of the symbol DCC as a possible reference to the "deleted in colon cancer" gene for two diseased: breast cancer and colon cancer. The number of occurrences and the matching score (0 to 1 low to high) is given after each extracted definition of the symbol. Thus, Table 2 shows how the symbol "DCC" is disambiguated in two contexts, one of breast cancer and the other of colon cancer. Although the symbol occurs twice as often in documents dealing with breast cancer, an embodiment of the invention allows us to recognize that DCC in the context of colon cancer stands for the "deleted in colon cancer" gene, but stands for "dextran coated charcoal" in the breast cancer context. Dextran coated charcoal assay is the preferred method used to quantify the presence of estrogen and progesterone receptors in breast cancer tissue. This makes the symbol DCC highly relevant to breast cancer, but not the gene DCC itself. By analyzing the definitions accompanying the symbol, we were able to give opposite, but correct, classifications for DCC in two different contexts. The results shown in Table 2 may be shown, for example, in the display 115 of the server 110 (Figure 1).

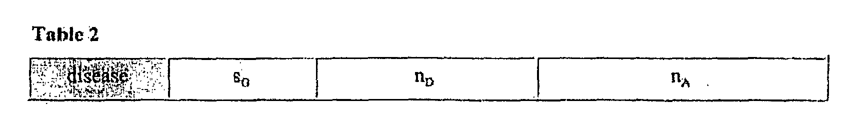

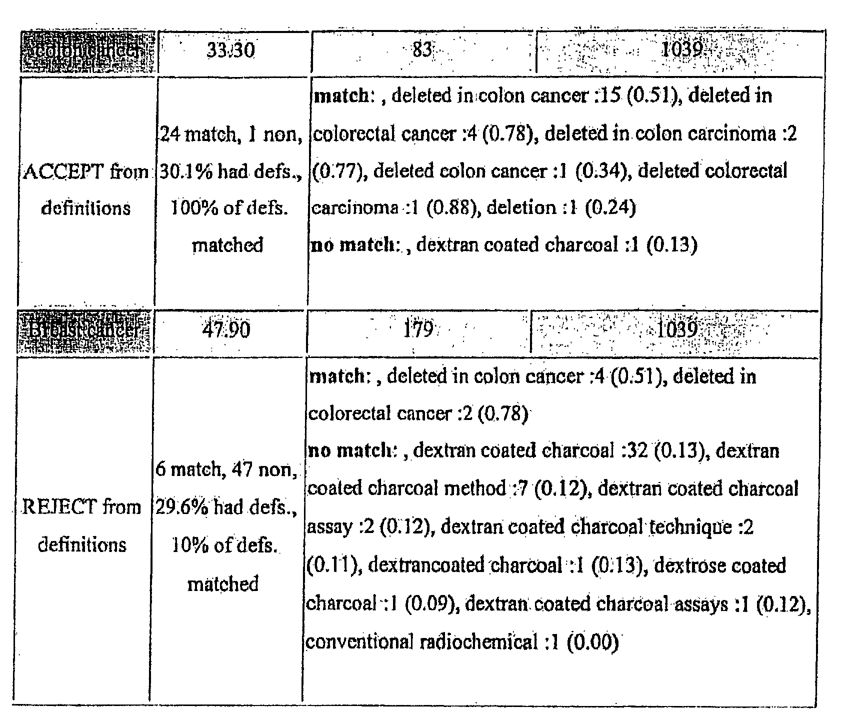

### Alternative features or other modifications

The various engines or modules discussed herein may be, for example, software, commands, data files, programs, code, modules, instructions, or the like, and may also include suitable mechanisms.

Reference throughout this specification to "one embodiment", "an embodiment", or "a specific embodiment" means that a particular feature, structure, or characteristic described in connection with the embodiment is included in at least one embodiment of the present invention. Thus, the appearances of the phrases "in one embodiment", "in an embodiment", or "in a specific embodiment" in various places throughout this specification are not necessarily all referring to the same embodiment. Furthermore, the particular features, structures, or characteristics may be combined in any suitable manner in one or more embodiments.

Other variations and modifications of the above-described embodiments and methods are possible in light of the foregoing teaching.

Further, at least some of the components of an embodiment of the invention may be implemented by using a programmed general purpose digital computer, by using application specific integrated circuits, programmable logic devices, or field programmable gate arrays, or by using a network of interconnected components and circuits. Connections may be wired, wireless, by modem, and the like.

It will also be appreciated that one or more of the elements depicted in the drawings/figures can also be implemented in a more separated or integrated manner, or even removed or rendered as inoperable in certain cases, as is useful in accordance with a particular application.

It is also within the scope of the present invention to implement a program or code that can be stored in a machine-readable medium to permit a computer to perform any of the methods described above.

Additionally, the signal arrows in the drawings/Figures are considered as exemplary and are not limiting, unless otherwise specifically noted. Furthermore, the term "or" as used in this disclosure is generally intended to mean "and/or" unless otherwise indicated. Combinations of components or actions will also be considered as being noted, where terminology is foreseen as rendering the ability to separate or combine is unclear.

As used in the description herein and throughout the claims that follow, "a", "an", and "the" includes plural references unless the context clearly dictates otherwise. Also, as used in the description herein and throughout the claims that follow, the meaning of "in" includes "in" and "on" unless the context clearly dictates otherwise.

The above description of illustrated embodiments of the invention, including what is described in the Abstract, is not intended to be exhaustive or to limit the invention to the precise forms disclosed. While specific embodiments of, and examples for, the invention are described herein for illustrative purposes, various equivalent modifications are possible within the scope of the invention, as those skilled in the relevant art will recognize.

These modifications can be made to the invention in light of the above detailed description. The terms used in the following claims should not be construed to limit the invention to the specific embodiments disclosed in the specification and the claims. Rather, the scope of the invention is to be determined entirely by the following claims, which are to be construed in accordance with established doctrines of claim interpretation.

## Claims

1. A method (200) of finding genes associated with a disease, the method comprising:
finding (205) all potential gene symbols;
folding (210) any aliases into at least one official gene symbol; and
computing (215) the relevance of each official symbol to the disease.

2. The method of claim 1, wherein the action of finding all potential gene symbols, comprises:
finding all potential gene symbols in a portion of an article.

3. The method of claim 2, wherein the portion is a title of the article.

4. The method of claim 2, wherein the portion is an abstract of the article.

5. The method of any preceding claim, wherein the action of folding any aliases into at least one official gene symbol includes:
if an alias symbol only one official symbol and the official symbol occurs independently, then replacing each mention of the alias symbol in a PMID/gene list.

6. The method of any of claims 1 to 4, wherein the action of folding any aliases into at least one official gene symbol includes:
if an alias symbol represents one or more official symbol, but only one of the official symbols occurs independently, then replacing the alias symbol in a PMID/gene list with the official symbol that occurs independently.

7. The method of any of claims 1 to 4, wherein the action of folding any aliases into at least one official gene symbol includes:
if an alias symbol represents one or more official symbol, but none of the official symbols occurs independently within a subset, then keeping the alias symbol in a PMID/gene list.

8. The method of any of claims 1 to 4, wherein the action of folding any aliases into at least one official gene symbol includes:
if an alias symbol represents more than one official symbol and at least two of these official symbols occur independently within a subset, then choosing an official symbol as a representation of the alias symbol based upon a syntactic analysis of text of abstracts or titles.

9. The method of any preceding claim, further comprising:
disambiguating a potential gene symbol.

10. An apparatus (100) for finding genes associated with a disease, the apparatus comprising:
a database (105) for storing information; and
a server (110) coupled to the database and configured to find all potential gene symbols in the stored information, to fold any aliases into official gene symbols; and to compute the relevance of each official symbol to the disease.
